**Europäisches Patentamt**

**European Patent Office**

**Office européen des brevets**

(19)

(11) Veröffentlichungsnummer : **0 172 896**
**B1**

(12) **EUROPÄISCHE PATENTSCHRIFT**

(45) Veröffentlichungstag der Patentschrift :
·10.02.88

(21) Anmeldenummer : 85901350.0

(22) Anmeldetag : 25.02.85

(86) Internationale Anmeldenummer :
PCT/DE 85/00058

(87) Internationale Veröffentlichungsnummer :
WO/8503886 (12.09.85 Gazette 85/20)

(51) Int. Cl.⁴ : **B 01 L   3/02**, B 01 L   3/00,
C 12 M   1/20, G 01 N 33/53

(54) **VERFAHREN UND EINRICHTUNG ZUM GLEICHZEITIGEN AUFBRINGEN EINER VIELZAHL VON FLÜSSIGKEITSPRO-BEN AUF EINEN OBJEKTTRÄGER.**

(30) Priorität : 29.02.84 DE 3407849

(43) Veröffentlichungstag der Anmeldung :
05.03.86 Patentblatt 86/10

(45) Bekanntmachung des Hinweises auf die Patenter-teilung : 10.02.88 Patentblatt 88/06

(84) Benannte Vertragsstaaten :
AT BE CH DE FR GB LI NL

(56) Entgegenhaltungen :
DE-A- 2 116 886
DE-A- 3 107 964

(73) Patentinhaber : NOSS, Gerhard
Bruchholtzweg 3
D-3006 Burgwedel (DE)

HÖFT, Alois
Elsternweg 4
D-3201 Algermissen (DE)

(72) Erfinder : NOSS, Gerhard
Bruchholtzweg 3
D-3006 Burgwedel (DE)
Erfinder : HÖFT, Alois
Elsternweg 4
D-3201 Algermissen (DE)

(74) Vertreter : Meyer, Ludgerus A. et al
Patentanwälte Holländer & Meyer Jungfernstieg 38
D-2000 Hamburg 36 (DE)

EP 0 172 896 B1

## Beschreibung

Die Erfindung betrifft ein Verfahren zum gleichzeitigen Aufbringen einer Vielzahl von Flüssigkeitsproben auf einen Objektträger, insbesondere von Seren auf einem mit Zellkulturen bewachsenen Objektträger, sowie eine Einrichtung zur Durchführung des Verfahrens.

Zur mikroskopischen Untersuchung von an Antigene einer Zellkultur angebundenen Antikörpern aus einem aufgebrachten Serum ist es bekannt, einen Objektträger der Standardgröße 26 × 76 mm oder größer mit aufgebrachten Zellkulturen zu versehen und diese mit Hilfe einer Pipette mit Serum zu impfen. Dazu werden vielfach Multipipetten verwendet, die das gleichzeitige Aufbringen einer Vielzahl von Serenproben ermöglichen. Mit Hilfe dieser Multipipetten können zuvor auch die Zellkulturen selbst auf den Objektträger aufgebracht werden.

Es erfordert hohe Konzentration und Geschicklichkeit, die Zellkulturen in einem relativ engen Raster auf einen Objektträger aufzubringen und diese dann jeweils zentral mit einem Serum zu impfen. Außerdem ergibt sich wegen ungleicher Höhe der Austrittsspitzen der Vielfachpipette eine ungleichmäßige Verteilung auf dem Objektträger. Zur Verhinderung des Verlaufs des Serums von einer Zellkultur zur nächsten ist daher bekannt, den Objektträger mit einer Schutzschicht vorzugsweise aus Teflon® mit einer Vielzahl von Ausnehmungen, in denen sich jeweils eine Zellkultur befindet, zu überziehen. Die Verwendung von mit einer Schutzschicht überzogenen Objektträgern erleichtert zwar die Handhabung und Sicherung gegen Verlauf, jedoch ist das Aufbringen der Schutzschicht auf dem Objektträger nicht von den Untersuchungslabors selbst möglich und daher sind teure bereits beschichtete Objektträger zu verwenden. Im Handel erhältliche Objektträger mit einer Schutzschicht weisen darüber hinaus den Nachteil auf, daß deren Schutzschicht gegenüber gewissen Reinigungsflüssigkeiten, insbesondere Aceton, empfindlich reagiert und daher nur beschränkt zu verwenden sind.

Die Verwendung von nicht mit einer Schutzschicht überzogenen Objektträgern setzt der Anwendung bei der obengenannten Untersuchung enge Grenzen, denn die Abstände der auf dem Objektträger aufgebrachten Zellkulturen sind zur Verhinderung des Verlaufs untereinander sehr groß zu wirtschaftlich ist.

Aus der US-A-3 776 699 ist zwar eine Anordnung bekannt, die ein regelmäßiges Aufbringen einer Flüssigkeit in kleinsten Mengen auf einen Objektträger gestattet. Da jedoch keine Schutzschicht auf dem Objektträger verwendet wid, ist hierbei das Problem des Verlaufs nicht verbessert. Das Aufbringen der Flüssigkeit erfolgt hier in nach oben offenen Vertiefungen. Da die einzelnen Tropfen somit aus einer bestimmten Höhe auf den Objektträger fallen, ist ein Verlauf der Tropfen nicht auszuschließen. Dies ist um so eher möglich, als die mit dieser Vorrichtung aufzubringende Mindestflüssigkeitsmenge 25 µl überschreitet.

In der US-A-3 356 462 ist eine Platte mit einer rasterförmigen Anordnung von Behältnissen angegeben, die zur Aufnahme von Flüssigkeiten zur Mikrotitration dient. Aus der DE-A-21 16 886 ist auch eine der Platte der US-A-3 356 462 ähnliche flexible Folie mit einer Vielzahl von durchlöcherten Behältnissen bekannt, die zur überführung einer Vielzahl von Flüssigkeitsproben in Behältnisse entsprechend der US-A-33 56 462 bei der Titration dient. Die Flexibilität der Folie sowie Wandungsstärke und Form der Durchtrittsöffnungen verhindern jedoch den Einsatz für die Zwecke der Erfindung.

Der Erfindung liegt die Aufgabe zugrunde, ein Verfahren zum gleichzeitigen Aufbringen einer Vielzahl von Flüssigkeitsproben auf einen Objektträger anzugeben, bei dem die Flüssigkeitsproben in geringem Abstand nebeneinander auf dem Objektträger angeordnet werden können, ohne daß ein Verlauf untereinander stattfindet. Es ist weiterhin Aufgabe der Erfindung, eine Einrichtung zur Durchführung des Verfahrens anzugeben, die einfach zu handhaben ist, billig in der Herstellung sowie den besonderen Bedürfnissen der Laboruntersuchung angepaßt ist. Es soll ferner ein Objektträger ver wendbar sein, der in einer Weise vorbereitet ist, daß eine Reihenuntersuchung von Seren schnell, genau und zuverlässig ausgeführt werden kann.

Die Aufgabe der Erfindung wird durch die in den Ansprüchen angegebene Erfindung gelöst. Vorteilhafte Weiterbildungen der Erfindung sind in Unteransprüchen, der Beschreibung und den Zeichnungen angegeben.

Das erfindungsgemäße Verfahren ermöglicht ein sehr schnelles und sicheres Aufbringen einer Vielzahl von Flüssigkeitsproben auf einen Objektträger, wie es insbesondere bei der Reihenuntersuchung von Blutseren auf Antikörperbildung wünschenswert ist. Die Verfahrensschritte der Erfindung sind ohne besondere Geschicklichkeit auszuführen, da die verwendete Trägerplatte eine Trichterfunktion hat und damit das genaue Zentrieren einer Vielfachpipette erleichtert. Zur Durchführung des Verfahrens ist ein beliebiger Objektträger zu verwenden, der keinerlei Schutzschicht aufweisen muß. Vorzugsweise kann bei der Durchführung der Erfindung jedoch ein Objektträger Verwendung finden, der bereits mit einer standardisierten Zellkultur belegt ist. In diesem Falle ist ein besonders schnelles Untersuchen der zu untersuchenden Blutseren möglich.

Die Erfindung wird nachstehend anhand eines Ausführungsbeispiels erläutert. Die Figuren zeigen besonders Darstellungen und Ausführungsformen der Erfindung.

Fig. 1 zeigt eine Einrichtung nach der Erfindung,

Fig. 2 einen Ausschnitt von Fig. 1 zur Verdeutlichung der Positionen der für des Verfahren verwendeten Einrichtungen,

Fig. 3 eine Darstellung des Austrittsvorgangs der Flüssigkeitsproben,

Fig. 4 eine Aufsicht auf eine Trägerplatte,

Fig. 5 eine Unteransicht eines Behältnisses,

Fig. 6 eine Seitenansicht eines Halters.

In medizinischen Untersuchungslabors ist die Untersuchung von Blutseren auf Antikörper Bestandteil der alltäglichen Arbeit. Die Bestimmung der Antikörperbildung erfolgt im allgemeinen u. a. mittels fluoreszenzmikroskopischer Techniken. Dazu werden Objektträger mit einer Zellkultur bewachsen, auf die dann Blutseren in der Menge von 10-20 µl aufgebracht werden. Nach Ablauf einer Inkubationszeit kann das Ergebnis bestimmt werden.

Beim erfindungsgemäßen Verfahren wird zunächst ein Objektträger, der für die gleichzeitige Untersuchung einer Vielzahl von Proben vorzugsweise eine Größe von 80 × 120 mm aufweist, zunächst mit einer Vielzahl von Zellkulturen belegt. Die Zellkultur kann entweder auf dem gesamten Objektträger gleichmäßig in einer dünnen Schicht aufgebracht sein, oder punktweise in gleichen Abständen. Die zweite Methode ist im allgemeinen wirtschaftlicher, da beim Aufbringen der Seren eine räumliche Trennung der verschiedenen Serenproben erforderlich ist, und in den Zwischenräumen das Vorhandensein von Zellkulturen daher überflüssig und unwirtschaftlich ist. Zum Aufbringen der Zellkulturen auf den Objektträger kann das erfindungsgemäße Verfahren ebenfalls mit Vorteil angewendet werden.

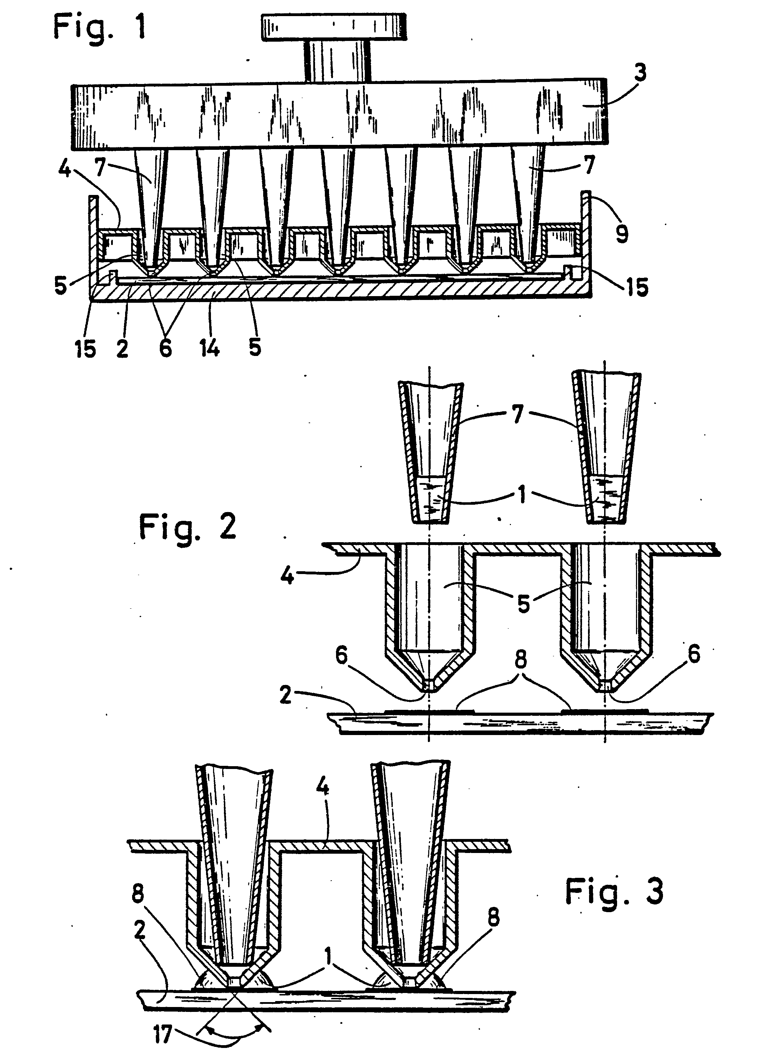

Fig. 1 zeigt die erfindungsgemäße Einrichtung zur Durchführung des Verfahrens. Der Objektträger 2 befindet sich dazu zwischen Stegen 15 auf der Bodenplatte 14 des kastenförmigen Halters 9. Der Objektträger 2 liegt damit unverrückbar innerhalb des Halters 9 fest. Auf den Objektträger 2 ist die weiter unten näher beschriebene Trichterplatte 4 aufgelegt. Diese Trichterplatte 4 enthält eine Vielzahl von nach unten enger werdenden trichterförmigen Behältnissen 5, deren in einer Spitze zulaufende Unterseiten auf dem Objektträger 2 aufliegen. Durch die dem Objektträger 2 zugekehrten Öffnungen 6 läßt sich mit Hilfe einer Vielfachpipette 3, deren flexibLe Austrittsspitzen 7 auf den Boden der Behältnisse 5 gebracht werden, durch Betätigen der Vielfachpipette durch jede der Auslauföffnungen 6 eine vorbestimmte Flüssigkeitsmenge hindurchdrücken. Die durch die Auslauföffnungen 6 hindurchgedrückte Flüssigkeitsmenge verteilt sich auf der Objektträgerplatte kreisförmig um die Auslauföffnungen 6. Die auf diese Weise auf einem Objektträger 2 aufgebrachten Zellkulturen wachsen nach Wegnehmen der Trichterplatte 4 in wenigen Tagen zur erforderlichen Größe heran.

Nach Reinigung der Trichterplatte 4 kann diese gegebenenfalls wieder verwendet werden, um mit Hilfe der Vielfachpipette 3 auf die auf der Trägerplatte vorhandenen Zellkulturen Blutseren zu übertragen.

Fig. 2 zeigt die übereinander dargestellte Anordnung von Objektträger 2, Trägerplatte 4 und Austrittsspitzen 7 der Vielfachpipette 3 in vergrößerter Darstellung. Die Austrittsspitzen 7 enthalten hierbei Flüssigkeitsproben 1, insbesondere ein Serum. Die Trägerplatte 2 weist Zellkulturen 8 auf, auf die die Auslauföffnungen 6 der Trägerplatte 4 aufsetzbar sind. Nach Aufsetzen der Trägerplatte 4 auf den Objektträger 2 und Einführen der Vielfachpipette 7 in die Behältnisse 5 der Trichterplatte 4 kann die Flüssigkeitsprobe 1 durch die Auslauföffnung 6 auf die Zellkultur 8 aufgebracht werden.

Fig. 3 zeigt eine Darstellung, die zeigt, wie eine Flüssigkeitsprobe 1 auf die Zellkultur 8 übertragen wird. Es ist dargestellt, daß die Flüssigkeitsprobe sich zwischen der Zellkultur 8 und der äußeren Wandung der Unterseite der Trichterplatte 4 befindet. Die Zellkultur 8 kann damit unmittelbar von dem Serum inkubiert werden. Durch Diffusion von Antikörpern, die im Serum enthalten sind, auf die Antigene innerhalb der Zellkulturen findet eine Inkubation statt, die eine Zeit zwischen etwa einer halben Stunde und drei Stunden benötigt. Diese Inkubation findet vorzugsweise durch gemeinsames Verbringen des Objektträgers 2 und der Trichterplatte 4 — durch die Halterung 9 fixiert — in einem Brutschrank statt. Auf diese Weise kann der Zustand, wie in Fig. 3 dargestellt, erhalten werden, nämlich daß die Flüssigkeitsproben sich zwischen der Zellkultur 8 und der Wandung der Trägerplatte 4 aufwölbt. Damit wird ein Verfließen des Serums zur benachbarten Zellkultur vermieden. Auf diese Weise kann andererseits der Abstand der benachbarten Zellkulturen sehr klein gehalten werden, ohne daß ein Übertrag von einer Zellkultur zur anderen zu befürchten ist. Nach Beendigung der Inkubation wird das überflüssige Serum angewaschen.

In einer besonderen Ausführungsform der Erfindung ist der verwendete Objektträger 2 bereits ein mit einer Standardzellkultur versehener Objektträger, der unter dafür erforderlichen Bedingungen, insbesondere der Einhaltung bestimmter Temperaturen, langfristig gelagert ist. Wenn auf einem derartigen Objektträger 2 Zellkulturen 8 im Raster, das der Trichterplatte 4 entspricht, angeordnet sind, ist es nicht notwendig, für schnelle Untersuchungen jeweils eine Zellkultur anwachsen zu lassen, die dann erst nach einer gewissen Zeit einsatzfähig ist.

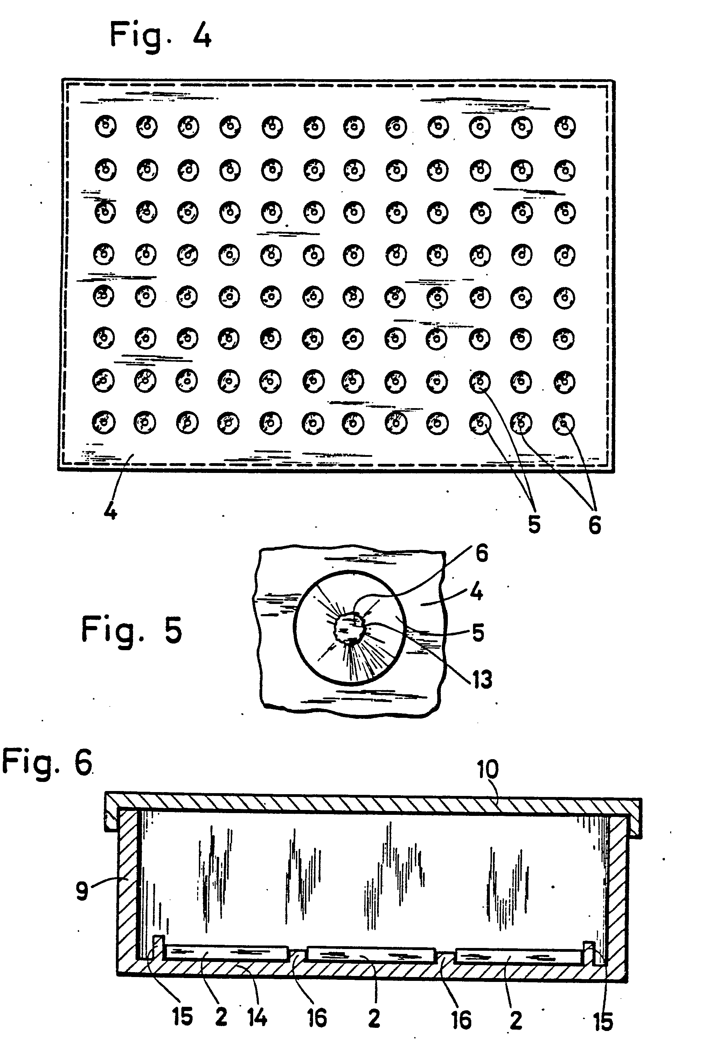

Fig. 4 zeigt eine Aufsicht auf eine Trichterplatte 4. Diese unterscheidet sich von der Anordnung nach der US-A-33 56 462 vor allem dadurch, daß die Behältnisse bei der Erfindung in ihrem unteren Scheitelpunkt durchbohrt sind. Die Behältnisse 5 dienen daher bei der Erfindung als Einführungstrichter für die verwendete Vielfachpipette 3. Die aus der DE-A-21 16 886 bekannte Folie ist für die Zwecke der Erfindung nicht geeignet, da sie durch ihre flexible Ausführung nicht gleichmäßig auf einer planen Unterlage aufliegen kann, so daß nur dort, wo die Vertiefungen der Folie Kontakt zur Unterlage hatten, Flüssigkeit austreten könnte. Bei der Verwendung der Folie zur überführung von Mikrovolumina in eine Platte nach der US-A-3 356 462 spielt dies jedoch keine Rolle. Ein gleichmäßiges zuverlässiges Aufbringen von Mikrovolumina auf eine plane Glasoberfläche ist daher mit einer derartigen Einrichtung nicht möglich.

Zu einer genauen Dosierung ist daher eine Trichterplatte mit einer für alle Behältnisse in gleicher Ebene sich befindenden Auslauföffnung unerläßlich. Dies wird insbesondere durch eine feste Trichterplatte erreicht. Durch eine besondere Gestaltung des Öffnungswinkels 17 des V-förmig zulaufenden unteren Abschnittes des im übrigen zylindrischen Behälters ergibt sich auf dem Objektträger 2 die in Fig. 3 dargestellte besondere Aufwölbung der Flüssigkeitsprobe 1. Der Winkel 17 sollte vorzugsweise mehr als 90° betragen. In einer praktischen Ausführungsform betrug der Winkel etwa 120°.

Die durch die Spitze der Behältnisse gehende Bohrung oder Stanzung weist etwa zylindrische Gestalt auf und ist in einer besonderen Ausführungsform der Erfindung an ihrer Außenseite nicht entgratet, sondern weist dort eine gewisse Rauhigkeit auf, um den seitlichen Austritt der Flüssigkeit 1 bei direktem Kontakt der Trichterplatte 4 mit dem Objektträger 2 zu erleichtern. Die Trichterplatte 4 weist in einer besonderen Ausführungsform der Erfindung ein Raster von 8 × 12 = 96 Behältnissen auf.

Fig. 5 zeigt den aufgerauhten Rand der Bohrung 13 auf der Unterseite des Behältnisses 5.

Um auch Objektträger kleinerer Größe in der Anordnung nach der Erfindung verwenden zu können, sind auf der Bodenplatte 14 des Halters 9 weitere Stege 16 vorhanden, die ein gegenseitiges Verschieben von kleineren Objektträgern 2 verhindern (Fig. 6). Wenn die Stege 15, die einen großen Objektträger seitlich begrenzen etwas höher ausgebildet sind als die weiteren Stege 16, kann der Halter 9 für beide Größen von Objektträgern verwendet werden. Bei Verwendung eines großen Objektträgers liegt dieser auf den weiteren Stegen 16 auf und wird durch die seitlich höheren Stege 15 begrenzt. Hingegen liegen kleinere Objektträger 2 zwischen den Stegen 16 bzw. 16 und 15.

Vorzugsweise ist ein deckelartiger Aufsatz 10 vorgesehen, der den Objektträger in dem Halter 9 bei Verbringen in den Brutschrank gegen äußere Einflüsse schützt.

Die Erfindung ist nicht auf die Anwendung in der medizinischen Untersuchungstechnik beschränkt, vielmehr lassen sich durch das erfindungsgemäße Verfahren auch z. B. Farbmarkierungen auf Leiterplatten in der Elektronikindustrie anbringen oder es können auch chemische Produkte örtlich erzeugt werden.

**Patentansprüche**

1. Verfahren zum gleichzeitigen Aufbringen einer Vielzahl von Flüssigkeitsproben (1) auf einem Objektträger (2), insbesondere von Seren auf einen mit Zellkulturen bewachsenen Objektträger (2), bei dem die Flüssigkeitsproben (1) mit Hilfe einer Vielfachpipette (3) auf den Objektträger (2) aufgebracht werden, dadurch gekennzeichnet, daß zunächst eine Trichterplatte (4) mit einer Vielzahl von in einer Ebene nebeneinander liegenden trichterförmigen Behältnissen (5) mit deren Auslauföffnungen (6) auf dem Objektträger (2) aufliegend diesem in fester Position zugeordnet wird, und daß dann die Flüssigkeitsproben (1) nach Einführen der Ausstrittsspitzen (7) der Vielfachpipette (3) in die Behältnisse (5) bis auf deren Grund unmittelbar über den Auslauföffnungen (6) durch die Auslauföffnungen (6) hindurch auf den Objektträger (2) aufgebracht werden.

2. Verfahren nach Anspruch 1, dadurch gekennzeichnet, daß der Objektträger (2) vor dem Aufbringen der Flüssigkeitsproben (1) mit einer Zellkultur (8) belegt wird.

3. Verfahren nach Anspruch 2, dadurch gekennzeichnet, daß die Trichterplatte (4) solange in ihrer Position zum Objektträger (2) belassen wird, bis eine ausreichende Durchdringung der Flüssigkeitsproben (1) mit der Zellkultur (8) stattgefunden hat.

4. Verfahren nach Anspruch 2, dadurch gekennzeichnet, daß die Flüssigkeitsproben (1) Blutseren sind.

5. Verfahren nach Anspruch 3 und 4, dadurch gekennzeichnet, daß Objektträger (2) und Trichterplatte (4) in Position zueinander belassen werden, bis die Kulturen (8) durch in den Seren vorhandene Moleküle inkubiert sind.

6. Einrichtung zum gleichzeitigen Aufbringen einer Vielzahl von Flüssigkeitsproben (1) auf einen Objektträger, dadurch gekennzeichnet, daß eine Trichterplatte mit einer Vielzahl von in einer Ebene nebeneinander liegenden trichterförmigen Behältnissen (5) vorgesehen ist, die von der oberen Fläche der Trichterplatte (4) aus im wesentlichen V-förmig zulaufend ausgebildet sind und in deren unteren Enden zylindrische Auslauföffnungen (6) vorgesehen sind, die auf dem in fester Position zur Trichterplatte angeordneten Objektträger aufliegen.

7. Einrichtung nach Anspruch 6, dadurch gekennzeichnet, daß der Rand (13) der Bohrungen an der Außenseite der Behältnisse (5) aufgerauht ist.

8. Einrichtung nach Anspruch 6 zum Aufbringen einer Flüssigkeitsprobe mit einem Volumen von 10-20 µl, dadurch gekennzeichnet, daß der Öffnungswinkel (17) der V-förmig zulaufenden unteren Enden der Behältnisse (5) größer als 90° beträgt.

9. Einrichtung nach Anspruch 6, dadurch gekennzeichnet, daß ein Halter (9) mit seitlichen Führungen (15, 16) zur Halterung des Objektträgers (2) und der Trichterplatte (4) vorgesehen ist, in den die Trichterplatte (4) auf den Objektträger (2) in seitlicher fixierter Zuordnung einlegbar ist.

10. Einrichtung nach Anspruch 9, dadurch gekennzeichnet, daß der Halter (9) kastenförmig ausgebildet ist.

11. Einrichtung nach Anspruch 9, dadurch gekennzeichnet, daß auf der Bodenplatte (14) des Halters (9) Stege (15) zur seitlichen Festlegung eines aufgelegten Objektträgers (2) vorgesehen sind.

12. Einrichtung nach Anspruch 11, dadurch gekennzeichnet, daß weitere Stege (16) auf der Bodenplatte (14) zur Unterteilung der Auflagefläche vorgesehen sind, um das Nebeneinanderliegen von mehreren gegenüber der Gesamtfläche der Bodenplatte (14) kleineren Objektträgern (2) zu ermöglichen.

13. Einrichtung nach Anspruch 6, dadurch gekennzeichnet, daß der Objektträger mit einer Vielzahl von seitlich beabstandeten Zellkulturen (8) belegt ist.

14. Einrichtung nach Anspruch 13, dadurch gekennzeichnet, daß der Abstand und die Anordnung der Zellkulturen (8) dem Abstand und der Anordnung der Auslauföffnungen (6) der Trichterplatte (4) entsprechen, so daß jeweils eine Auslauföffnung (6) einer Zellkultur (8) zugeordnet ist.

## Claims

1. A method of simultaneously applying a plurality of liquid samples (1) to an object slide (2), more particularly for applying serum samples to an object slide (2) coated with cell cultures, in which method the liquid samples (1) are applied to the object slide (2) by means of a multiple pipette (3), characterised in that a funnel plate (4) containing a plurality of funnel-shaped containers (5) disposed side by side in one plane is firstly associated with the object slide (2) in a fixed position with the container outlet apertures (6) resting on the object slide (2), and then after the outlet tips (7) of the multiple pipette (3) have been introduced into the containers (5) as far as the base thereof directly above the outlet apertures (6) the liquid samples are applied through the outlet apertures (6) on to the object slide (2).

2. A method according to claim 1, characterised in that before the liquid samples (1) are applied the object slide (2) is coated with a cell culture (8).

3. A method according to claim 2, characterised in that the funnel plate (4) is left in its position relative to the object slide (2) until there has been adequate penetration of the liquid samples (1) by the cell culture (8).

4. A method according to claim 2, characterised in that the liquid samples (1) are blood serums.

5. A method according to claims 3 and 4, characterised in that the object slide (2) and the funnel plate (4) are left in position relative to one another until the cultures (6) have incubated through molecules present in the serums.

6. Apparatus for simultaneously applying a plurality of liquid samples (1) to an object slide, characterised in that a funnel plate is provided, which has a plurality of funnel-shaped containers (5) which are disposed side by side in one plane and which, from the top surface of the funnel plate (4), are constructed to taper substantially in the form of a V, and in the bottom ends of which cylindrical outlet apertures (6) are provided which rest on the object slide disposed in a fixed position relative to the funnel plate.

7. Apparatus according to claim 6, characterised in that the edge (13) of the bores is roughened on the outside of the containers (5).

8. Apparatus according to claim 6 for applying a liquid sample with a volume of 10-20 μl, characterised in that the aperture angle (17) of the V-shaped tapering bottom ends of the containers (5) is more than 90°.

9. Apparatus according to claim 6, characterised in that a holder (9) with lateral guides (15, 16) is provided to hold the object slide (2) and the funnel plate (4), into which holder the funnel plate (4) is insertable on to the object slide (2) in a laterally fixed association.

10. Apparatus according to claim 9, characterised in that the holder (9) is of box-shaped construction.

11. Apparatus according to claim 9, characterised in that webs (15) are provided on the baseplate (14) of the holder (9) in order laterally to fix an object slide (2) applied thereto.

12. Apparatus according to claim 11, characterised in that further webs (16) are provided on the baseplate (14) in order to subdivide the support surface in order to allow a plurality of object slides (2) which are relatively small in comparison with the total area of the baseplate (14) to be disposed side by side.

13. Apparatus according to claim 6, characterised in that the object slide is coated with a plurality of laterally spaced cell cultures (8).

14. Apparatus according to claim 13, characterised in that the spacing and arrangement of the cell cultures (8) correspond to the spacing and arrangement of the outlet apertures (6) of the funnel plate (4) so that an outlet aperture (6) is associated with a cell culture (8) in each case.

## Revendications

1. Procédé pour déposer simultanément une pluralité d'échantillons de liquide (1) sur un porte-objet (2), en particulier une pluralité de sérums sur un porte-objet (2) recouvert de cultures cellulaires dans lequel les échantillons de liquide (1) sont déposés sur les porte-objets (2) à l'aide d'une pipette multiple (3), caractérisé en ce que d'abord une plaque à entonnoirs (4) comportant une pluralité de récipients en forme d'entonnoirs (5) juxtaposés les uns aux autres dans un plan et dont les orifices de décharge (6) reposent, le porte-objet (2), est montée en position fixe sur celui-ci, et en ce qu'ensuite, après introduction des becs débordants (7) de la pipette multiple dans les récipients (5) jusqu'au fond de ceux-ci, les échantillons de liquide (1) sont déposés directement sur le porte-objet (2) par l'intermédiaire des orifices de décharge (6) et à travers ceux-ci.

2. Procédé selon la revendication 1, caractérisé en ce que, préalablement au dépôt des échantillons de liquide (1), le porte-objet (2) est recouvert d'une culture cellulaire (8).

3. Procédé selon la revendication 2, caractérisé en ce que la plaque à entonnoirs (4) est laissée dans sa position vis-à-vis du porte-objet (2), jusqu'à ce que s'opère une imprégnation suffisante des échantillons de liquide (1) avec la culture cellulaire (8).

4. Procédé selon la revendication 2, caractérisé en ce que les échantillons de liquide (1) sont des sérums sanguins.

5. Procédé selon les revendications 3 et 4, caractérisé en ce que le porte-objet (2) et la plaque à entonnoirs (4) sont laissés en position l'un vis-à-vis de l'autre, jusqu'à ce que les cultures (8) soient incubées par des molécules présentes dans les sérums.

6. Dispositif pour déposer simultanément une pluralité d'échantillons de liquide (1) sur un porte-objet, caractérisé en ce qu'il est prévu une plaque à entonnoirs (4) comportant une pluralité de récipients en forme d'entonnoirs (5) juxtaposés les uns aux autres dans un plan, qui, à partir de la surface supérieure de la plaque à entonnoirs (4), présentent une configuration se prolongeant sensiblement en forme de V, et dans les extrémités inférieures desquels sont ménagés des orifices de décharge (6) cylindriques qui reposent sur le porte-objet disposée en position fixe vis-à-vis de la plaque à entonnoirs.

7. Dispositif selon la revendication 6, caractérisé en ce que le bord (13) des perçages est rendu rugueux au niveau de la face extérieure des récipients (5).

8. Dispositif selon la revendication 6 pour déposer un échantillon de liquide ayant un volume de 10-20 µl, caractérisé en ce que l'angle d'orifice (17) des extrémités inférieures se prolongeant en forme de V, des récipients (5) est supérieur à 90°.

9. Dispositif selon la revendication 6, caractérisé en ce qu'il est prévu un support (9) pourvu de glissières latérales (15, 16) pour supporter le porte-objet (2) et la plaque à entonnoirs (4), dans lequel la plaque à entonnoirs (4) posée sur le porte-objet (2) est apte à être logée suivant un montage à fixation latérale.

10. Dispositif selon la revendication 9, caractérisé en ce que le support (9) est conçu en forme de caisson.

11. Dispositif selon la revendication 9, caractérisé en ce que sur la plaque de base (14) du support (9), sont prévues des nervures (15) en vue de la fixation latérale d'un porte-objet (2) posé.

12. Dispositif selon la revendication 11, caractérisé en ce que d'autres nervures (16) sont prévues sur la plaque de base (14) en vue de subdiviser la surface d'appui, afin de permettre la juxtaposition de plusieurs porte-objets (2) plus petits par rapport à la surface totale de la plaque de base (14).

13. Dispositif selon la revendication 6, caractérisé en ce que le porte-objet est recouvert d'une pluralité de cultures cellulaires (8) espacées latéralement les unes des autres.

14. Dispositif selon la revendication 13, caractérisé en ce que l'espacement et la disposition des cultures cellulaires (8) coïncident avec l'espacement et avec la disposition des orifices de décharge (6) de la plaque à entonnoirs (4) de façon qu'à chaque orifice de décharge (6) corresponde une culture cellulaire (6).

0 172 896

Fig. 1

Fig. 2

Fig. 3

# Fig. 4

4   5   6

# Fig. 5

6
4
5
13

# Fig. 6

10

9

15   2   14   16   2   16   2   15